(19) Europäisches Patentamt
European Patent Office
Office européen des brevets

(11) Veröffentlichungsnummer: **0 309 906 A1**

(12) # EUROPÄISCHE PATENTANMELDUNG

(21) Anmeldenummer: **88115561.8**

(22) Anmeldetag: **22.09.88**

(51) Int. Cl.⁴: **C07D 307/28 , C07C 47/20 , C07C 47/21**

(30) Priorität: **30.09.87 DE 3732951**

(43) Veröffentlichungstag der Anmeldung: **05.04.89 Patentblatt 89/14**

(84) Benannte Vertragsstaaten: **BE DE FR GB NL**

(71) Anmelder: **BASF Aktiengesellschaft**
**Carl-Bosch-Strasse 38**
**D-6700 Ludwigshafen(DE)**

(72) Erfinder: **Hoelderich, Wolfgang, Dr.**
**Mannheimer Strasse 18 c**
**D-6710 Frankenthal(DE)**
Erfinder: **Schwarzmann, Matthias, Dr.**
**Carl-Bosch-Strasse 54**
**D-6703 Limburgerhof(DE)**

(54) **Verfahren zur Herstellung von Dihydrofuranen und alpha, beta-ungesättigten Aldehyden/Ketonen.**

(57) Die Anmeldung betrifft ein Verfahren zur Herstellung von Dihydrofuranen der Formel (I) und $\alpha,\beta$-ungesättigte Aldehyden bzw. Ketonen der Formel (II)

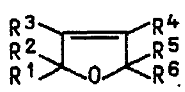
(I),
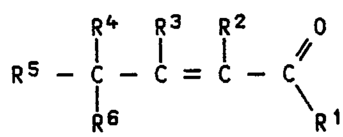
(II)

in der $R^1$ bis $R^6$ gleich oder verschieden sein können und Wasserstoff, Alkyl- bzw. Alkenyl- mit 1 bis 6 Kohlenstoffatomen, Alkoxyl-, Aryl-, Aralkyl- oder Alkylarylresten bedeuten, bei dem man ungesättigte Diole der Formel (III)

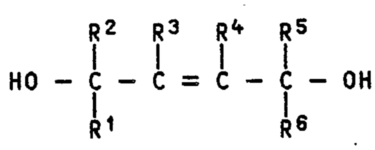
(III)

in der $R^1$ bis $R^6$ obige Bedeutung haben, in Gegenwart von Zeolithen als Katalysatoren umsetzt.

EP 0 309 906 A1

Xerox Copy Centre

**Verfahren zur Herstellung von Dihydrofuranen und α,β-ungesättigten Aldehyden/Ketonen**

Gegenstand der Erfindung ist ein Verfahren zur Herstellung von Dihydrofuranen und α,β-ungesättigten Aldehyden/Ketonen aus ungesättigten Diolen in Gegenwart von Katalysatoren.

Es ist bekannt, daß man Tetrahydrofuran (THF) aus 1,4-Butandiol in flüssiger Phase in Gegenwart von sauren Homogenkatalysatoren wie $H_2SO_4$, $H_3PO_4$, Toluolsulfonsäure mit hohen Ausbeuten von etwa 95 % erhalten kann. Ein Nachteil ist hierbei die Abtrennung des Katalysators. Bei gleichzeitiger Dehydrierung kann man 4,5-Dihydrofuran in 86 %iger Ausbeute erhalten. Diese Verbindung kann man auch durch Umsetzung von α-Acetylenalkoholen in Gegenwart basischer Katalysatoren, wie Natriumamid, mit etwa 50 % Ausbeute herstellen. Auch sulfonsäuregruppenhaltige Kationenaustauscher-Harze können für diese intramolekulare Dehydratisierung eingesetzt werden. Hierbei erzielt man 92 bis 95 % Ausbeute (Houben-Weyl, Bd. Sauerstoffverbindungen I, Teil 3, Georg-Thieme-Verlag, 1965).

Die Umsetzung von Diolen an Aluminosilikatzeolithen vom X- und Y-Typ ist ebenfalls bekannt (A. Molnar et al., Proc. ZEOCAT Symp. Siofok, Ungarn, 1985, Acta Physica et Chemica Szegediensis, Szeged, 1985, S. 5). Neben der gewünschten Dehydratisierung tritt hierbei auch selektivitätsmindernde Fragmentierung des C-Gerüstes auf. Die Tetrahydrofurane kann man durch partielle Dehydrierung in die Dihydrofurane mit mäßigen Ausbeuten überführen. Die Umwandlung ungesättigter Diole zu α,β-ungesättigten Aldehyden/Ketonen ist nicht beschrieben.

Es wurde nun gefunden, daß man Dihydrofurane der Formel (I) und α,β-ungesättigte Aldehyde bzw. Ketone der Formel (II)

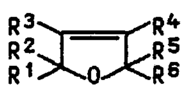 (I), 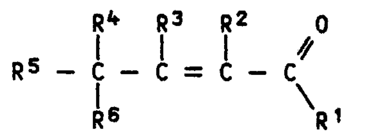 (II)

in der $R^1$ bis $R^6$ gleich oder verschieden sein kann und Wasserstoff, Alkyl-bzw. Alkenyl- mit 1 bis 6 Kohlenstoffatomen, Alkoxyl-, Aryl-, Aralkyl-oder Alkylarylresten bedeuten, in guten Ausbeuten erhält, wenn man ungesättigte Diole der Formel (III)

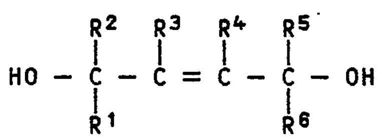 (III)

in der $R^1$ bis $R^6$ obige Bedeutung haben, in Gegenwart von Zeolithen als Katalysatoren umsetzt. Die Doppelbindung kann im Ring der Dihydrofurane der Formel (I) verschoben sein.

Ein Vorteil des erfindungsgemäßen Verfahrens besteht darin, daß man durch geeignete Wahl von Temperatur und Belastung das Produktspektrum steuern kann.

Als Reste $R^1$ bis $R^6$ kommen Wasserstoff sowie geradkettige oder verzweigte Alkyl- oder Alkenylreste mit 1 bis 12, insbesondere 1 bis 8, vorzugsweise 1 bis 4 Kohlenstoffatomen in Betracht.

Alkylreste sind z.B. Methyl-, Ethyl-, Propyl-, n-Butyl-, i-Butyl-, Pentyl-, Hexyl-, Octyl- oder Decylreste. Alkenylreste sind z.B. Propenyl-, Butenyl-, Hexenyl- oder Octenylreste.

Als Cycloalkylreste für $R^1$ bis $R^6$ kommen z.B. Cyclopentyl-, Cyclohexyl-, Cycloheptyl-, Cyclopentenyl- oder Cyclohexenylreste in Betracht.

Aromatische Reste für $R^1$ bis $R^6$ sind z.B. Phenyl-, Benzyl-, Toluyl-, Phenylethyl-, p-Methylbenzyl- oder p-Propylphenylreste.

Als Rest $R^1$ bis $R^6$ kommen z.B. Methoxy-, Ethoxy-, n-/i-Propoxy, n-/i-/t-Butoxy-, Hexoxyreste in Betracht.

Als Katalysatoren werden bei dem erfindungsgemäßen Verfahren Zeolithe, insbesondere in der aciden Form verwendet. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2 (Ullmanns Encyclopädie der technischen Chemie, 4. Auflage, Band 24, Seite 575, 1983). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydratation durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt. So bilden bei der Mordernit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe. In diese Gruppe von Zeolithen gehören auch die sogenannten "ultrastabilen" Zeolithe des Faujasit-Typs, d.h. dealuminierte Zeolithe. Verfahren zur Herstellung solcher Zeolithe sind beschrieben in "Catalysis by Zeolites", Band 5 aus "Studies in Surface Science and Catalysis" ed. B. Imelik et al. Elsevier Scientific Publishing Comp. 1980, S. 203 und "Crystal Structures of Ultra-stable Faujasites" Advances in Chemistry Series Nr. 101, American Chemical Society Washington, DC, S. 226 ff (1971) und in US-PS 4 512 961.

Besonders vorteilhaft sind Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und denen vom Typ X oder Y liegen.

Diese Zeolithe können unterschiedliche chemische Zusammensetzungen aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220 °C unter autogenem Druck hergestellt. Hierzu gehören auch die isotaktischen Zeolithe nach EP 34 727 und EP 46 504. Die erhaltenen Aluminosilikatzeolithe enthalten je nach Wahl der Einsatzstoffmengen ein $SiO_2/Al_2O_3$-Verhältnis von 10 bis 40.000. Aluminosilikatzeolithe können auch in etherischem Medium, wie Diethylenglykoldimethylether, in alkoholischem Medium, wie Methanol bzw. 1,4-Butandiol oder in Wasser synthetisiert werden.

Borosilikatzeolithe kann man z.B. bei 90 bis 200 °C unter autogenem Druck synthetisieren, indem man eine Borverbindung wie $H_3BO_3$ mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetraminlösung mit und insbesondere ohne Alkali- oder Erdalkalizusatz zur Reaktion bringt. Auch die oben erwähnten isotaktischen Zeolithe sind verwendbar. Die Borosilikatzeolithe können statt in wäßriger Aminlösung auch in etherischer Lösung, z.B. Diethylenglykoldimethylether oder in alkoholischer Lösung, z.B. 1,6-Hexandiol hergestellt werden.

Eisensilikatzeolithe kann man aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220 °C unter autogenem Druck erhalten.

Zu den erfindungsgemäß verwendbaren siliciumreichen Zeolithen ($SiO_2/Al_2O_3 \geqq 10$) gehören auch die sog. ZSM-Typen, Ferrierit, NU-1 und Silicalit®, ein Molekularsieb, ein sog. Silica Polymorph.

Die so hergestellten Alumino-, Boro- und Eisensilikatzeolithe können nach ihrer Isolierung, Trocknung bei 100 bis 160 °C, vorzugsweise bei 110 °C und Calcinierung bei 450 bis 550 °C, vorzugsweise bei 500 °C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:5, bevorzugt 75:25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 °C/16 h getrocknet und bei 500 °C/16 h calciniert.

Man erhält auch vorteilhafte Katalysatoren, wenn der isolierte Alumino-bzw. Borosilikatzeolith direkt nach der Trocknung verformt und erst nach der Verformung einer Calcinierung unterworfen wird. Die

hergestellten Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegen die Zeolithe aufgrund der Herstellungsweise nicht in der katalytisch aktiven, aciden H-Form vor, sondern in der Na-Form, dann kann diese durch Ionenaustausch mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eine durch Koksabscheidung bedingte Desaktivierung eintritt, empfiehlt es sich, die Zeolithe durch Abtrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, kann es vorteilhaft sein, die Zeolithe zu modifizieren. Eine geeignete Modifizierung der Katalysatoren besteht z.B. darin, daß man die unverformten oder verformten Zeolithe mit Metallsalzen durch Ionenaustausch oder durch Imprägnierung dotiert. Als Metallsalze sind die Salze der Alkalimetalle wie Li, Cs, K, der Erdalkalimetalle wie Mg, Ca, Sr, der Metalle der 3., 4. und 5. Hauptgruppe wie Al, Ga, Ge, Sb, Pb, Bi, der Übergangsmetalle der 4. bis 8. Nebengruppe wie Ti, Zr, V, Nb, Cr, Mo, W, Mn, Re, Fe, Ru, Os, Co, Rh, Sr, Ni, Pd, Pt, der Übergangsmetalle der 1. und 2. Nebengruppe wie Cu, Ag, Zn, sowie der Seltene Erdmetalle wie La, Ce, Pr, Nd, Fr, Yb und U geeignet.

Zweckmäßigerweise führt man die Dotierung so durch, daß man die verformten Zeolithe in einem Steigrohr vorlegt und bei 20 bis 100 °C eine wäßrige oder ammoniakalische Lösung eines Halogenids oder eines Nitrats der genannten Metalle überleitet. Ein derartiger Ionenaustausch kann an der Wasserstoff-, Ammonium- und Alkaliform der Zeolithe vorgenommen werden. Eine weitere Möglichkeit der Metallaufbringung auf die Zeolithe besteht darin, daß man das zeolithische Material mit einem Halogenid, einem Nitrat oder einem Oxid der genannten Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2 \times 3\ H_2O$ oder $Ni(NO_3)_2 \times 6\ H_2O$ oder $Ce(NO_3)_3 \times 6\ H_2O$ oder $La(NO_3)_2 \times 6\ H_2O$ oder $Cs_2CO_3$ oder $H_2WO_4$ in Wasser löst. Mit dieser Lösung werden die verformten oder unverformten Zeolithe etwa 30 Minuten getränkt. Die eventuell überstehende Lösung wird am Rotationsverdampfer von Wasser befreit. Danach wird der getränkte Zeolith bei etwa 150 °C getrocknet und bei etwa 550 °C calciniert. Dieser Tränkvorgang kann mehrmals hintereinander vorgenommen werden, um den gewünschten Metallgehalt einzustellen.

Es ist auch möglich, eine wäßrige $Ni(CO_3)_2$-Lösung oder ammoniakalische $Pd(NO_3)_2$-Lösung herzustellen und darin die reinen pulverförmigen Zeolithe bei 40 bis 100 °C unter Rühren etwa 24 h aufzuschlämmen. Nach Abfiltrieren, Trocknen bei etwa 150 °C und Calcinierung bei etwa 500 °C kann das gewonnene zeolithische Material mit oder ohne Bindemittel zu Strängen, Pellets oder Wirbelgut weiterverarbeitet werden.

Ein Ionenaustausch der in der H-Form, Ammonium-Form oder Alkali-Form vorliegenden Zeolithe kann so vorgenommen werden, daß man die Zeolithe in Strängen oder Pellets in einer Kolonne vorlegt und darüber eine wäßrige $Ni(NO_3)_2$-Lösung oder ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei etwa 150 °C getrocknet und bei etwa 550 °C calciniert. Bei manchen metalldotierten Zeolithen, z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft so vor, daß man Zeolithe in Pulverform mit 1 n Phosphorsäure 1 Stunde bei 80 °C behandelt. Nach der Behandlung wird mit Wasser gewaschen, bei 110 °C/16 Stunden getrocknet und bei 500 °C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80 °C mit einer 3 bis 25 gew.%igen, insbesondere 12 bis 20 gew.%igen wäßrigen Salzsäure. Anschließend werden die so behandelten Zeolithe mit Wasser gewaschen, getrocknet und bei 400 °C bis 500 °C calciniert.

Eine besondere Ausführungsform für die Säurebehandlung besteht darin, daß man das zeolithische Material vor seiner Verformung bei erhöhter Temperatur mit 0,001 bis 2 n, insbesondere 0,05 bis 0,5 n

Flußsäure behandelt, beispielsweise durch Erhitzen unter Rückfluß über einen Zeitraum von 0,5 bis 5, insbesondere 1 bis 3 Stunden. Nach Isolierung durch Abfiltrieren und Auswaschen des zeolithischen Materials wird dieses zweckmäßig bei Temperaturen von 100 bis 160 °C getrocknet und bei Temperaturen von 450 bis 600 °C calciniert. Man kann das zeolithische Material aber auch nach Verformung mit Bindemittel bei erhöhter Temperatur, zweckmäßig bei 50 bis 90 °C, insbesondere 60 bis 80 °C, über einen Zeitraum von 0,5 bis 5 h mit 12 bis 20 gew.%iger Salzsäure behandeln. Anschließend wird das zeolithische Material ausgewaschen und zweckmäßig bei Temperaturen von 100 bis 160 °C getrocknet und bei Temperaturen von 450 bis 600 °C calciniert. Einer HF-Behandlung kann sich auch eine HCl-Behandlung anschließen.

Die Zeolithe können auch durch Aufbringung von Phosphorverbindungen, wie Trimethoxiphosphat, Trimethoxiphosphin, primärem, sekundärem oder tertiärem Natriumphosphat modifiziert werden. Hierbei werden die Zeolithe in Strang-, Tabletten- oder Wirbelgut-Form mit wäßriger $H_3PO_4$-Lösung getränkt, bei 110 °C getrocknet und bei 500 °C calciniert.

Die beschriebenen Katalysatoren können wahlweise als 2- bis 4 mm-Stränge oder als Tabletten mit 3 bis 5 mm Durchmesser oder als Splitt mit Teilchengrößen von 0,1 bis 0,5 mm oder als Wirbelkontakt eingesetzt werden.

Das erfindungsgemäße Verfahren wird bevorzugt in der Gasphase bei 100 bis 500 °C, insbesondere 200 bis 400 °C, und einer Belastung von WHSV = 0,1 bis 20 $h^{-1}$, insbesondere 0,5 bis 5 $h^{-1}$ (g Ausgangsstoff/g Katalysator und Stunde) ausgeführt. Die Reaktion kann im Festbett oder im Wirbelbett ausgeführt werden. Es ist auch möglich, die Reaktion in der Flüssigphase (Suspension-, Riesel- oder Sumpffahrweise) bei Temperaturen zwischen 50 und 200 °C durchzuführen.

Das Verfahren wird zweckmäßig bei Normaldruck aber auch bei vermindertem oder erhöhtem Druck, vorzugsweise kontinuierlich aber auch diskontinuierlich durchgeführt.

Schwerflüchtige oder feste Ausgangsstoffe werden in gelöster Form, z.B. in THF-, Toluol- oder Petrolether-Lösung eingesetzt. Allgemein ist eine Verdünnung der Ausgangsstoffe mit Lösungsmitteln oder mit Inertgasen wie $N_2$, Ar, $H_2O$-Dampf möglich. In besonderen Fällen kann man auch $O_2$ verwenden.

Nach der Umsetzung wird das Endprodukt durch übliche Verfahren, z.B. durch Destillation, aus dem Reaktionsgemisch isoliert; nichtumgesetzte Ausgangsstoffe werden gegebenenfalls in die Umsetzung zurückgeführt. Es ist vorteilhaft, die gasförmigen Reaktionsprodukte sofort in eine Trennung einzubringen und anschließend, z.B. in einer Fraktionierkolonne, in die Einzelkomponenten zu zerlegen.

Beispiele 1 bis 11

Die Reaktionen in der Gasphase werden unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangsprodukte erfolgt gaschromatographisch.

Die für das erfindungsgemäße Verfahren eingesetzten Katalysatoren sind:

Katalysator A

Der Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50:50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew.% $SiO_2$ und 2,3 Gew.% $B_2O_3$.

Mit diesem Material werden durch Verformen mit einem Verformungshilfsmittel 2 mm-Stränge hergestellt, die bei 110 °C/16 h getrocknet und bei 500 °C/24 h calciniert werden.

Katalysator B

Katalysator B wird erhalten, indem man den Borosilikatzeolith von Katalysator A mit Boehmit im Gewichtsverhältnis 60:40 zu 2 mm-Strängen verformt, bei 110 °C/16 h trocknet und bei 500 °C/24 h calciniert. Diese Stränge werden mit wäßriger $H_2WO_4$-Lösung imprägniert, bei 130 °C/2 h getrocknet und bei 540 °C/2 h calciniert. Der W-Gehalt beträgt 3,1 Gew.%.

Katalysator C

Ein Aluminosilikatzeolith vom Pentasil-Typ wird unter hydrothermalen Bedingungen bei autogenem Druck und 150 °C aus 65 g hochdispersem $SiO_2$, 20,3 g $Al_2(SO_4)_3$ x 18 $H_2O$ in 1 kg einer wäßrigen 1,6-Hexandiamin-Lösung (Mischung 50:50 Gew.%) in einem Rührautoklaven hergestellt. Nach Abfiltrieren und Auswaschen wird das kristalline Reaktionsprodukt bei 110 °C/24 h getrocknet und bei 500 °C/24 h calciniert. Dieser Aluminosilikatzeolith enthält 91,6 Gew.% $SiO_2$ und 4,6 Gew.% $Al_2O_3$.

Der Katalysator wird mit einem Verformungshilfsmittel zu 2 mm-Strängen verformt, bei 110 °C/16 h getrocknet und bei 500 °C/24 h calciniert.

Katalysator D

Der Eisensilikatzeolith des Pentasil-Typs wird unter hydrothermalen Bedingungen bei autogenem Druck und 165 °C aus 273 g Wasserglas, gelöst in 253 g einer wäßrigen 1,6-Hexandiamin-Lösung (Mischung 50:50 Gew.%) und 31 g Eisensulfat, gelöst in 21 g 96 %iger Schwefelsäure und 425 g Wasser in einem Rührautoklaven während 4 Tagen synthetisiert. Der Zeolith wird abfiltriert, bei 110 °C/24 h getrocknet und bei 500 °C/24 h calciniert. Man erhält einen Eisensilikatzeolithen mit einem $SiO_2/Fe_2O_3$-Verhältnis von 17,7 und einem $Na_2O$-Gehalt von 1,2 Gew.%. Der Katalysator wird mit hochdispersem $SiO_2$ im Gewichtsverhältnis 80:20 zu 2,5 mm-Strängen verstrangt, bei 110 °C/16 h getrocknet und bei 500 °C/24 h calciniert.

Katalysator E

Katalysator E erhält man, indem man die Stränge des Katalysators B mit einer wäßrigen Lösung aus Cernitrat statt $H_2WO_4$-Lösung imprägniert, danach bei 130 °C/2 h trocknet und bei 540 °C/2 h calciniert. Der Ce-Gehalt beträgt 2,5 Gew.%.

Die mit diesen Katalysatoren erzielten Versuchsergebnisse und Versuchsbedingungen sind in der Tabelle zusammengefaßt.

Die Ergebnisse der Beispiele laut Tabelle zeigen, daß man durch geeignete Wahl des Katalysators und der Reaktionsbedingungen die Zusammensetzung des Reaktionsproduktes aus Dihydrofuranen und/oder $\alpha,\beta$-ungesättigten Aldehyden/Ketonen aus ungesättigten Diolen beeinflußen kann. Die Verbindungen können überraschend wahlweise durch einfache Temperatur- und Belastungsveränderung aus ein und demselben Ausgangsmaterial erhalten werden und dies in sehr hohen Ausbeuten, ohne daß Nebenreaktionen an der Doppelbindung des Einsatzstoffes stattfinden. Ebenso überraschend ist auch die hohe Standzeit des Katalysators; durch Oligomerisation bzw. Polymerisation und Polykondensation zu höhermolekularen Verbindungen hatte man mit schneller Desaktivierung rechnen müssen.

Tabelle

| Diole ----→ Dihydropyrane + α,β-ungesättigte Aldehyde + $H_2O$ | | | | | | | | | | | |
|---|---|---|---|---|---|---|---|---|---|---|---|
| Beispiel | 1 | 2 | 3 | 4 | 5 | 6 | 7 | 8 | 9 | 10 | 11 |
| Einsatzstoff | 1,4-Dihydrobuten-2,3 | | | | | | | 1,4-Dihydroxy-2-methylbuten-2,3 | | | |
| Katalysator | A | A | A | C | D | B | E | A | A | D | B |
| Temperatur [°C] | 200 | 300 | 400 | 400 | 400 | 200 | 400 | 300 | 400 | 400 | 200 |
| WHSV [$h^{-1}$] | 2,5 | 2,5 | 2,5 | 2,5 | 2,5 | 2,5 | 2,5 | 2,2 | 2,2 | 2,2 | 2,0 |
| Umsatz [%] | 100 | 100 | 100 | 100 | 100 | 100 | 100 | 100 | 100 | 100 | 100 |
| Selektivität [%] | | | | | | | | | | | |
| Dihydropyran | 87,5 | 41,9 | 8,5 | 2,5 | 4,4 | 73,8 | 5,6 | - | - | - | - |
| α,β-ungesättigter Aldehyd | 10,8[1)] | 57,4[1)] | 91,0[1)] | 87,7[1)] | 90,7[1)] | 20,4[1)] | 92,3[1)] | 92,8[2)] | 98,2[2)] | 94,2[2)] | 83,5[2)] |

1) Crotonaldehyd
2) Tiglinaldehyd

## Ansprüche

1. Verfahren zur Herstellung von Dihydrofuranen der Formel (I) und α,β-ungesättigte Aldehyden bzw. Ketonen der Formel (II)

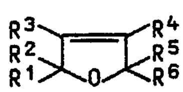 (I), 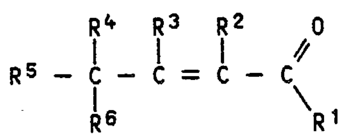 (II)

in der $R^1$ bis $R^6$ gleich oder verschieden sein kann und Wasserstoff, Alkyl- bzw. Alkenyl- mit 1 bis 6 Kohlenstoffatomen, Alkoxyl-, Aryl-, Aralkyl-oder Alkylarylresten bedeuten, dadurch gekennzeichnet, daß man ungesättigte Diole der Formel (III)

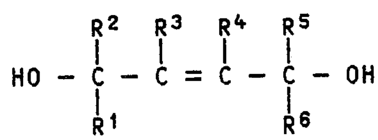 (III)

in der $R^1$ bis $R^6$ obige Bedeutung haben, in Gegenwart von Zeolithen als Katalysatoren umsetzt.

2. Verfahren nach Anspruch 1, dadurch gekennzeichnet, daß man 1,4-Butendiol umsetzt.

3. Verfahren nach Anspruch 1 und 2, dadurch gekennzeichnet, daß man als Katalysatoren Zeolithe vom Typ der L-, Y- oder X-Zeolithe verwendet.

4. Verfahren nach Anspruch 1 und 2, dadurch gekennzeichnet, daß man als Katalysatoren Zeolithe des Pentasiltyps verwendet.

5. Verfahren nach Anspruch 1 und 2, dadurch gekennzeichnet, daß man als Katalysatoren Aluminiumsilikatzeolithe verwendet.

6. Verfahren nach Anspruch 1 und 2, dadurch gekennzeichnet, daß man als Katalysatoren Borosilikatzeolithe verwendet.

7. Verfahren nach Anspruch 1 und 2, dadurch gekennzeichnet, daß man als Katalysatoren Eisensilikatzeolithe verwendet.

8. Verfahren nach Anspruch 1 bis 7, dadurch gekennzeichnet, daß man als Katalysatoren mit Alkali- und/oder Erdalkali- und/oder Übergangs- und/oder Seltenen Erd- und/oder Edelmetallen dotierte Zeolithe verwendet.

9. Verfahren nach Anspruch 1 bis 8, dadurch gekennzeichnet, daß man die Umsetzung in der Gasphase ausführt.

Europäisches Patentamt

# EUROPÄISCHER RECHERCHENBERICHT

Nummer der Anmeldung

EP 88115561.8

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| Y | DE - A1 - 2 509 968 (BASF)<br>* Beispiel 3 *<br>-- | 1 | C 07 D 307/28<br>C 07 C 47/20<br>C 07 C 47/21 |
| Y | CHEMICAL ABSTRACTS, Band 84, Nr. 13, 29. März 1976, Columbus, Ohio, USA<br>ARESHIDZE, KH. I.; CHIVADZE, G. O. " Tetrahydrofuran"<br>Seite 511, Spalte 2, Zusammenfassung Nr. 89 981a<br>& Otkrytiya, Izobret., Prom. Obraztsy, Tovarnye Znaki 1975, 52(45), 184<br>-- | 1 | |
| A | DE - A1 - 2 834 038 (MITSUBISHI)<br>* Anspruch 1 *<br>-- | 1 | RECHERCHIERTE SACHGEBIETE (Int. Cl.4) |
| A | FR - B1 - 2 295 037 (GENERAL ELECTRIC)<br>* Anspruch 1 *<br>-- | 1 | C 07 D 307/00<br>C 07 C 47/00 |
| A | US - A - 4 117 016 (HUGHES)<br>* Beispiel 1 *<br>-- | 1 | |

Der vorliegende Recherchenbericht wurde für alle Patentansprüche erstellt.

| Recherchenort | Abschlußdatum der Recherche | Prüfer |
|---|---|---|
| WIEN | 15-11-1988 | HAMMER |

KATEGORIE DER GENANNTEN DOKUMENTEN

- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

Europäisches Patentamt

# EUROPÄISCHER RECHERCHENBERICHT

Nummer der Anmeldung

- 2 -

EP 88115561.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 101, Nr. 25, 17. Dez. 1984, Columbus, Ohio, USA<br>POLIEVKA, MILAN; JEGOROV, ALEXANDR; UHLAR, LADISLAV; MACHO, VENDELIN "Hydroformylation of propene catalyzed with carbonylchlorobis (triphenylphosphine)rhodium(I)." Seite 719, Spalte 2, Zusammenfassung Nr. 229 940h<br>& Collect. Czech. Chem. Commun 1984, 49 (7), 1677-9<br>-- | 1 | |
| A | CHEMICAL ABSTRACTS, Band 90, Nr. 19, 7. Mai 1979, Columbus, Ohio, USA<br>TANABE, YASUO "Cyclic ethers"<br>Seite 592, Spalte 2, Zusammenfassung Nr. 151 964b<br>& Japan Tokkyo Koho 78 43 505<br>-- | 1 | |
| A | CHEMICAL ABSTRACTS, Band 90, Nr. 17, 23. April 1979, Columbus, Ohio, USA<br>TANABE, YASUO "Cyclic ethers"<br>Seite 499, Spalte 2, Zusammenfassung Nr. 137 659z<br>& Japan Tokkyo Koho 78 43 506<br>---- | 1 | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 15-11-1988 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument